# EUROPEAN PATENT SPECIFICATION

(11) **EP 2 189 163 B1**
(45) Date of publication and mention of the grant of the patent: **13.11.2013**
(21) Application number: 07785405.7
(22) Date of filing: 02.08.2007
(51) Int. Cl.: A23L 1/30, A61K 9/14, A61K 35/74, A61K 36/77, A61K 36/42, A61P 3/10

(54) **A COMPOSITION FOR TREATMENT OF DIABETES MELLITUS AND A PREPARATION AND AN USE THEREOF**
ZUSAMMENSETZUNG ZUR BEHANDLUNG VON DIABETES MELLITUS UND ZUBEREITUNG UND VERWENDUNG DAFÜR
COMPOSITION POUR LE TRAITEMENT DU DIABÈTE SUCRÉ ET SA PRÉPARATION ET SON UTILISATION

(30) Priority: 10.05.2007 CN 200710104381
(43) Date of publication of application: 26.05.2010
(73) Proprietor: Beijing DFJX New Technology Co., Ltd., Yizhuang Economic-Technological Development Area Beijing 100176 (CN)
(72) Inventor: Zhang, Xiaoyong, Zoucheng City, Shandong Province 273500 (CN)
(74) Representative: Moore, Michael Richard
(86) International application number: PCT/CN2007/070409
(87) International publication number: WO 2008/138191

(56) References cited:
- CN-A- 1 234 266
- CN-A- 1 579 234
- US-A- 5 086 043
- DATABASE WPI Week 200366 Thomson Scientific, London, GB; AN 2003-690274 XP002594273 & CN 1 397 293 A (LI C) 19 February 2003 (2003-02-19)
- DATABASE EMBASE [Online] ELSEVIER SCIENCE PUBLISHERS, AMSTERDAM, NL; 21 July 2005 (2005-07-21), CHEN J -G ET AL: "Effects of sugar-removed pumpkin zymotic powders in preventing and treating the increase of blood glucose in alloxan-induced diabetic mice" XP002594268 Database accession no. EMB-2005501394 & CHINESE JOURNAL OF CLINICAL REHABILITATION 20050721 CN, vol. 9, no. 27, 21 July 2005 (2005-07-21), pages 94-95, ISSN: 1671-5926
- DATABASE WPI Week 200143 Thomson Scientific, London, GB; AN 2001-398872 XP002594269 & CN 1 287 007 A (MEIYAN BLUE GREEN ALGAE CO LTD MEIXIAN C) 14 March 2001 (2001-03-14)
- MANI U V ET AL: "Studies on the long-term effect of spirulina supplementation on serum lipid profile and glycated proteins in NIDDM patients" JOURNAL OF NUTRACEUTICALS, FUNCTIONAL AND MEDICAL FOODS 2000 US LNKD- DOI:10.1300/J133V02N03_03, vol. 2, no. 3, 2000, pages 25-32, XP09136928 ISSN: 1089-4179
- DATABASE MEDLINE [Online] US NATIONAL LIBRARY OF MEDICINE (NLM), BETHESDA, MD, US; June 2004 (2004-06), GUO JIEWEN ET AL: "[Pharmacological mechanism of Semen Litchi on antagonizing insulin resistance in rats with type 2 diabetes]" XP002594271 Database accession no. NLM15524300 & ZHONG YAO CAI = ZHONGYAOCAI = JOURNAL OF CHINESE MEDICINAL MATERIALS JUN 2004 LNKD- PUBMED:15524300, vol. 27, no. 6, June 2004 (2004-06), pages 435-438, ISSN: 1001-4454
- DATABASE WPI Week 200512 Thomson Scientific, London, GB; AN 2005-102656 XP002594272 & CN 1 537 601 A (YUTAI SCI & TECH CO LTD) 20 October 2004 (2004-10-20)

## Description

### Field of the Invention

The present invention relates to a composition for treating diabetes and a preparation method thereof as well as use thereof.

### Background

In the past half century, various diabetics have nearly spread around each country and each region in the world. Diabetes is regarded as an internal medicine common disease, which is substantially characterized by persistent hyperglycemia, and the pathogenesis has not been definitely defined around the world currently. Diabetes is not a disease which is caused by single reason and single pathogenesis but the general name of a group of metabolic disease which is caused by inadequate insulin or low insulin effect for different reasons, wherein, the sugar metabolic disturbance is the essential pathologic reaction in the clinic. Diabetes can be generally divided into primary diabetes and secondary diabetes, wherein, secondary diabetes accounts for about 5% of the diabetes, which is generally named as insulin-dependent diabetes mellitus (IDDM), namely, type I diabetes; and the diabetic has little or no insulin: insulin injection is necessary to maintain diabetics life, otherwise, the diabetics suffer from the diabetic ketoacidosis and even quickly die. Primary diabetes accounts for about 95% of the diabetes, which is generally named as noninsulin-dependent diabetes mellitus, namely, type II diabetes; the disease onset is slow, and the onset age is after middle age usually but in any age at some times; self-engendered insulin level can normally increase or decrease, or secretion peak is delayed; about 60% diabetics is high weight or fat, and has hyperinsulinemia and insulin resistance; and most diabetics orally take hypoglycemic agent to control blood sugar, however, some of the diabetics also need insulin to control blood sugar, in particular to the II diabetics who are not fat. There are more than dozens kinds of medicaments for treating diabetes from home and abroad at the present, objectively speaking: the effects of the Chinese hypoglycemic agent and the Western hypoglycemic agent for treating type II diabetes are generally good and basically the same.

Thomson Scientific WPI database, Week 200366, AN 2003-690274, XP 002594273 & CN 1397293 discloses a health-care food for diabetics prepared from pumpkin powder, pumpkin flower powder and ginger powder.

Elsevier Science Publishers Embase Database, Chen J-G et al. "Effects of sugar-removed pumpkin zymotic powders in preventing and treating the increase of blood glucose in alloxan-induced diabetic mice", XP 002594268 discloses pumpkin zymotic powders for preventing and treating the increase of blood glucose in alloxan-induced diabetic mice.

Thomson Scientific WPI database, Week 200143, AN 2001-398872, XP 002594269 & CN 1287007 discloses a freeze-dried powder of spirulina in relation to blood sugar levels.

Mani U V *et al.* "Studies on the long-term effect of spirulina supplementation on serum lipid profile and glycated proteins in NIDDM patients" discloses the effect of supplementation with spirulina tablets on patients with non-insulin dependent diabetes mellitus.

US 5086043 discloses a pharmaceutical composition comprising oleanolic acid, saponins of Litchi and kuguasu for treating and preventing diabetes.

Medline database, XP 002594271, PUBMED: 15524300, Guo Jiewen *et al.* "Pharmacological mechanism of Semen Litchi on antagonizing insulin resistance in rats with type 2 diabetes" discloses an effect of Litchi seed extracts on antagonizing insulin resistance in rats with type 2 diabetes.

Thomson Scientific WPI database, Week 200512, AN 2005-102656, XP 002594272 & CN 1537601 discloses a health-care food comprising balsam pear for diabetes mellitus patients.

### Summary of the Invention

The present invention provides a composition for treating diabetes, comprising the following active components based on the parts by weight:
10-100 parts of Pumpkin powder;
6-200 parts of spirulina; and
2-90 parts of Lychee Seed.

Preferably, the composition further comprises 10-100 parts of balsam pear powder.

Preferably, the composition comprises the following active components based on the parts by weight:
10-60 parts of Pumpkin powder;
6-80 parts of spirulina;
2-60 parts of Lychee Seed; and
10-60 parts of balsam pear powder.

The invention further provides a method for preparing the composition, comprising steps of grinding and mixing the active components.

The invention further relates to use of the medicament for treating the diabetes.

The invention realizes the technical effects as follows:
By taking the composition, the blood sugar concentration of diabetics can be effectively controlled, the complicating diseases of diabetics can be avoided, and the health of diabetics can be protected.

### Detailed Description of the Invention

### Example 1

Firstly, the fresh pumpkin which is planted in accordance with the standard plant soil, plant water source and plant environment for planting green products and is free of any three wastes pollution source is cut into flakiness with equal size by a cutting tool, is naturally cooled under sterile circumstances and is dried into dried pumpkin after being normally processed; the dried pumpkin is cut into small pieces with size of about 1.5x1.5cm; the small pieces are dried in a sterilizing way to have standard colour and lustre with a high-temperature sterilizing drier under the standard sterilizing and drying temperature; the dried pumpkin is naturally cooled in sterile space; the dried pumpkin is sterilized with a sterile grinder into small pieces with size of about 1cmx1.5cm; and the small pieces are processed into pumpkin fine powder with size of Phi 0.2 mm to be spare by a professional procedure and device. Secondly, the fresh balsam pear which is planted in accordance with the standard plant soil, plant water source and plant environment for planting green products and is free of any three wastes pollution source is cut into flakiness with equal size by a cutting tool, is naturally cooled under the sterile circumstances and is dried into dried balsam pear after being normally processed; the dried balsam pear is cut into small pieces with size of about 1.2x1.5cm; the balsam pear is dried in a sterilizing way to have standard colour and lustre with a high-temperature sterilizing drier under the standard sterilizing and drying temperature; the dried balsam pear is naturally cooled in sterile space; the dried balsam pear is sterilized with a sterile grinder into small pieces with size of about 1.5x1.5cm; and the small pieces are processed into balsam pear fine powder with size of Phi 0.1 mm to be spare in a combination way by a professional procedure and device. Thirdly, the natural spirulina which is processed in a way of standard sterilization is processed into gain powder with size of Phi 0.1mm to be spare in a combination way by the same sterile fine grinder; and the spirulina which is processed in a way of standard sterilization is processed into gain powder with size of Phi 0.1mm so as to be spare in a combination way by the same sterile fine grinder. Finally, the skin and the flesh of the fresh lychee is skinned to remain the seed, the seed is dried by a standard sterilizing device and grinded into grains with size of Phi 0.5cm, and the dried seed is processed into lychee seed fine powder with size of Phi 0.15mm to be spare in a combination way according to different dosages after being processed in a way of standard sterilization.

### Example 2

Aiming at the levis diabetics in the type II diabetics, the dosage and the taking mode of breakfast on an empty stomach at every morning are prepared by dosing the powdery pumpkin fine powder, the balsam pear fine powder, the spirulina fine powder and the lychee seed fine powder which are prepared by the example 1 under sterile environment in the follows ways:
1. Adding the spirulina fine powder 8mg and the lychee seed fine powder 2mg into the pumpkin fine powder 14g to form type A breakfast for the levis diabetics in the type II diabetics, namely, preparing into soupy breakfast for food therapy on an empty stomach with 150ml boiled water under 80 °C 20 minutes before breakfast;
2. Adding the spirulina fine powder 10mg and the lychee seed fine powder 3mg into the pumpkin fine powder 14.5g to form type B breakfast for the levis diabetics in the type II diabetics, namely, preparing into soupy breakfast for food therapy on an empty stomach with 150ml boiled water under 80 °C 20 minutes before breakfast;
3. Adding the spirulina fine powder 12mg and the lychee seed fine powder 4mg into the pumpkin fine powder 15g to form type C breakfast for the levis diabetics in the type II diabetics, namely, preparing into soupy breakfast for food therapy on an empty stomach with 150ml boiled water under 80 °C 20 minutes before breakfast;
4. Adding the spirulina fine powder 14mg and the lychee seed fine powder 5mg into the pumpkin fine powder 15.5g to form type D breakfast for the levis diabetics in the type II diabetics, namely, preparing into soupy breakfast for food therapy on an empty stomach with 150ml boiled water under 80 °C 20 minutes before breakfast;
5. Adding the spirulina fine powder 16mg and the lychee seed fine powder 6mg into the pumpkin fine powder 16g to form type H breakfast for the levis diabetics in the type II diabetics, namely, preparing into soupy breakfast for food therapy on an empty stomach with 150ml boiled water under 80 °C 20 minutes before breakfast;
6. Adding the spirulina fine powder 18mg and the lychee seed fine powder 7mg into the pumpkin fine powder 16.5g to form type F breakfast for the levis diabetics in the type II diabetics, namely, preparing into soupy breakfast for food therapy on an empty stomach with 150ml boiled water under 80 °C 20 minutes before breakfast;
7. Adding the spirulina fine powder 20mg and the lychee seed fine powder 8mg into the pumpkin fine powder 17g to form type G breakfast for the levis diabetics in the type II diabetics, namely, preparing into soupy breakfast for food therapy on an empty stomach with 150ml boiled water under 80 °C 20 minutes before breakfast;
8. Adding the spirulina fine powder 20mg and the lychee seed fine powder 9mg into the pumpkin fine powder 17.2g to form type H breakfast for the levis diabetics in the type II diabetics, namely, preparing into soupy breakfast for food therapy on an empty stomach with 150ml boiled water under 80 °C 20 minutes before breakfast;
9. Adding the spirulina fine powder 20mg and the lychee seed fine powder 10mg into the pumpkin fine powder 17.4g to form type J breakfast for the levis diabetics in the type II diabetics, namely, preparing into soupy breakfast for food therapy on an empty stomach with 150ml boiled water under 80 °C 20 minutes before breakfast;

### Example 3

Aiming at the middle diabetics in the type II diabetics, the dosage and the taking mode of breakfast on an empty stomach at every morning are prepared by dosing the powdery pumpkin fine powder, the spirulina fine powder and the lychee seed fine powder which are prepared by the example 1 under sterile environment in the follows ways:
1. Adding the spirulina fine powder 22mg and the lychee seed fine powder 12mg into the pumpkin fine powder 17.5g to form type A breakfast for the middle diabetics in the type II diabetics, namely, preparing into soupy breakfast for food therapy on an empty stomach with 150ml boiled water under 80 °C 20 minutes before breakfast;
2. Adding the spirulina fine powder 24mg and the lychee seed fine powder 13mg into the pumpkin fine powder 18g to form type B breakfast for the middle diabetics in the type II diabetics, namely, preparing into soupy breakfast for food therapy on an empty stomach with 150ml boiled water under 80 °C 20 minutes before breakfast;
3. Adding the spirulina fine powder 26mg and the lychee seed fine powder 14mg into the pumpkin fine powder 18.5g to form type C breakfast for the middle diabetics in the type II diabetics, namely, preparing into soupy breakfast for food therapy on an empty stomach with 150ml boiled water under 80 °C 20 minutes before breakfast;
4. Adding the spirulina fine powder 28mg and the lychee seed fine powder 15mg into the pumpkin fine powder 19g to form type D breakfast for the middle diabetics in the type II diabetics, namely, preparing into soupy breakfast for food therapy on an empty stomach with 150ml boiled water under 80 °C 20 minutes before breakfast;
5. Adding the spirulina fine powder 30mg and the lychee seed fine powder 16mg into the pumpkin fine powder 19.5g to form type E breakfast for the middle diabetics in the type II diabetics, namely, preparing into soupy breakfast for food therapy on an empty stomach with 150ml boiled water under 80 °C 20 minutes before breakfast;
6. Adding the spirulina fine powder 32mg and the lychee seed fine powder 17mg into the pumpkin fine powder 20g to form type F breakfast for the middle diabetics in the type II diabetics, namely, preparing into soupy breakfast for food therapy on an empty stomach with 150ml boiled water under 80 °C 20 minutes before breakfast;
7. Adding the spirulina fine powder 34mg and the lychee seed fine powder 18mg into the pumpkin fine powder 20.5g to form type G breakfast for the middle diabetics in the type II diabetics, namely, preparing into soupy breakfast for food therapy on an empty stomach with 150ml boiled water under 80 °C 20 minutes before breakfast.
8. Adding the spirulina fine powder 34.5mg and the lychee seed fine powder 19mg into the pumpkin fine powder 20.7g to form type H breakfast for the middle diabetics in the type II diabetics, namely, preparing into soupy breakfast for food therapy on an empty stomach with 150ml boiled water under 80 °C 20 minutes before breakfast;
9. Adding the spirulina fine powder 35mg and the lychee seed fine powder 20mg into the pumpkin fine powder 20.9g to form type J breakfast for the middle diabetics in the type II diabetics, namely, preparing into soupy breakfast for food therapy on an empty stomach with 150ml boiled water under 80 °C 20 minutes before breakfast;

### Example 4

Aiming at the severe diabetics in the type II diabetics, the dosage and the taking mode of breakfast on an empty stomach at every morning are prepared by dosing the powdery pumpkin fine powder, the spirulina fine powder and the lychee seed fine powder which are prepared by the example 1 under sterile environment in the follows ways:
1. Adding the spirulina fine powder 36mg and the lychee seed fine powder 21mg into the pumpkin fine powder 21g to form type A breakfast for the severe diabetics in the type II diabetics, namely, preparing into soupy breakfast for food therapy on an empty stomach with 150ml boiled water under 90 °C 20 minutes before breakfast;
2. Adding the spirulina fine powder 38mg and the lychee seed fine powder 21mg into the pumpkin fine powder 21.5g to form type B breakfast for the severe diabetics in the type II diabetics, namely, preparing into soupy breakfast for food therapy on an empty stomach with 150ml boiled water under 90 °C 20 minutes before breakfast;
3. Adding the spirulina fine powder 40mg and the lychee seed fine powder 22mg into the pumpkin fine powder 22g to form type C breakfast for the severe diabetics in the type II diabetics, namely, preparing into soupy breakfast for food therapy on an empty stomach with 150ml boiled water under 90 °C 20 minutes before breakfast;
4. Adding the spirulina fine powder 42mg and the lychee seed fine powder 22.5mg into the pumpkin fine powder 22.5g to form type D breakfast for the severe diabetics in the type II diabetics, namely, preparing into soupy breakfast for food therapy on an empty stomach with 150ml boiled water under 90 °C 20 minutes before breakfast;
5. Adding the spirulina fine powder 44mg and the lychee seed fine powder 21mg into the pumpkin fine powder 23g to form type E breakfast for the severe diabetics in the type II diabetics, namely, preparing into soupy breakfast for food therapy on an empty stomach with 150ml boiled water under 90 °C 20 minutes before breakfast;
6. Adding the spirulina fine powder 46mg and the lychee seed fine powder 24mg into the pumpkin fine powder 23.5g to form type F breakfast for the severe diabetics in the type II diabetics, namely, preparing into soupy breakfast for food therapy on an empty stomach with 150ml boiled water under 90 °C 20 minutes before breakfast;
7. Adding the spirulina fine powder 48mg and the lychee seed fine powder 27mg into the pumpkin fine powder 24g to form type G breakfast for the severe diabetics in the type II diabetics, namely, preparing into soupy breakfast for food therapy on an empty stomach with 150ml boiled water under 90 °C 20 minutes before breakfast.
8. Adding the spirulina fine powder 48.2mg and the lychee seed fine powder 30mg into the pumpkin fine powder 24.2g to form type H breakfast for the severe diabetics in the type II diabetics, namely, preparing into soupy breakfast for food therapy on an empty stomach with 150ml boiled water under 90 °C 20 minutes before breakfast;
9. Adding the spirulina fine powder 48.5mg and the lychee seed fine powder 33mg into the pumpkin fine powder 24.4g to form type J breakfast for the severe diabetics in the type II diabetics, namely, preparing into soupy breakfast for food therapy on an empty stomach with 150ml boiled water under 90 °C 20 minutes before breakfast;

### Example 5

Aiming at the levis diabetics in the type II diabetics, the preparation, the dosage and the taking mode of snacks for balancing non-fasting blood sugar at every morning are prepared by dosing the prepared lychee seed fine powder, the spirulina fine powder and the balsam pear fine powder under sterile environment in the follows ways:
1. Adding the spirulina fine powder 15mg and the balsam pear fine powder 3g into the lychee seed fine powder 2.5g, and evenly mixing the mixture to form 1g/piece troche or 0.5-1g/gian capsule, namely, forming the snacks for balancing non-fasting blood sugar for the levis diabetics in the type II diabetics, wherein, the snacks are taken 5 minutes within the type A breakfast, the lunch and the dinner with warm water under 60 °C;
2. Adding the spirulina fine powder 25mg and the balsam pear fine powder 3.5g into the lychee seed fine powder 3.5g, and evenly mixing the mixture to form 1g/piece troche or 0.5-1g/gian capsule, namely, forming the snacks for balancing non-fasting blood sugar for the levis diabetics in the type II diabetics, wherein, the snacks are taken 5 minutes within the type B breakfast, the lunch and the dinner with warm water under 60 °C;
3. Adding the spirulina fine powder 35mg and the balsam pear fine powder 4g into the lychee seed fine powder 4.5g, and evenly mixing the mixture to form 1g/piece troche or 0.5-1g/gian capsule, namely, forming the snacks for balancing non-fasting blood sugar for the levis diabetics in the type II diabetics, wherein, the snacks are taken 5 minutes within the type C breakfast, the lunch and the dinner with warm water under 60 °C;
4. Adding the spirulina fine powder 45mg and the balsam pear fine powder 4.5g into the lychee seed fine powder 5.5g, and evenly mixing the mixture to form 1g/piece troche or 0.5-1g/gian capsule, namely, forming the snacks for balancing non-fasting blood sugar for the levis diabetics in the type II diabetics, wherein, the snacks are taken 5 minutes within the type D breakfast, the lunch and the dinner with warm water under 60 °C;
5. Adding the spirulina fine powder 55mg and the balsam pear fine powder 5g into the lychee seed fine powder 6.5g, and evenly mixing the mixture to form 1g/piece troche or 0.5-1g/gian capsule, namely, forming the snacks for balancing non-fasting blood sugar for the levis diabetics in the type II diabetics, wherein, the snacks are taken 5 minutes within the type E breakfast, the lunch and the dinner with warm water under 60 °C;
6. Adding the spirulina fine powder 65mg and the balsam pear fine powder 5.5g into the lychee seed fine powder 7.5g, and evenly mixing the mixture to form 1g/piece troche or 0.5-1g/gian capsule, namely, forming the snacks for balancing non-fasting blood sugar for the levis diabetics in the type II diabetics, wherein, the snacks are taken 5 minutes within the type F breakfast, the lunch and the dinner with warm water under 60 °C.
7. Adding the spirulina fine powder 75mg and the balsam pear fine powder 6g into the lychee seed fine powder 8.5g, and evenly mixing the mixture to form 1g/piece troche or 0.5-1g/gian capsule, namely, forming the snacks for balancing non-fasting blood sugar for the levis diabetics in the type II diabetics, wherein, the snacks are taken 5 minutes within the type G breakfast, the lunch and the dinner with warm water under 60 °C;
8. Adding the spirulina fine powder 77mg and the balsam pear fine powder 6.5g into the lychee seed fine powder 8.7g, and evenly mixing the mixture to form 1g/piece troche or 0.5-1g/gian capsule, namely, forming the snacks for balancing non-fasting blood sugar for the levis diabetics in the type II diabetics, wherein, the snacks are taken 5 minutes within the type H breakfast, the lunch and the dinner with warm water under 60 °C;
9. Adding the spirulina fine powder 79mg and the balsam pear fine powder 6.9g into the lychee seed fine powder 8.9g, and evenly mixing the mixture to form 1g/piece troche or 0.5-1g/gian capsule, namely, forming the snacks for balancing non-fasting blood sugar for the levis diabetics in the type II diabetics, wherein, the snacks are taken 5 minutes within the type J breakfast, the lunch and the dinner with warm water under 60 °C;

### Example 6

Aiming at the middle diabetics in the type II diabetics, the preparation, the dosage and the taking mode of snacks for balancing non-fasting blood sugar at every morning are prepared by dosing the prepared lychee seed fine powder, the spirulina fine powder and the balsam pear fine powder under sterile environment in the follows ways:
1. Adding the spirulina fine powder 25mg and the balsam pear fine powder 4g into the lychee seed fine powder 9.5g, and evenly mixing the mixture to form 1g/piece troche or 0.5-1g/gian capsule, namely, forming the snacks for balancing non-fasting blood sugar for the middle diabetics in the type II diabetics, wherein, the snacks are taken 5 minutes within the type A breakfast, the lunch and the dinner with warm water under 60 °C;
2. Adding the spirulina fine powder 35mg and the balsam pear fine powder 4.5g into the lychee seed fine powder 9.7g, and evenly mixing the mixture to form 1g/piece troche or 0.5-1g/gian capsule, namely, forming the snacks for balancing non-fasting blood sugar for the middle diabetics in the type II diabetics, wherein, the snacks are taken 5 minutes within the type B breakfast, the lunch and the dinner with warm water under 60 °C;
3. Adding the spirulina fine powder 45mg and the balsam pear fine powder 5g into the lychee seed fine powder 9.9g, and evenly mixing the mixture to form 1g/piece troche or 0.5-1g/gian capsule, namely, forming the snacks for balancing non-fasting blood sugar for the middle diabetics in the type II diabetics, wherein, the snacks are taken 5 minutes within the type C breakfast, the lunch and the dinner with warm water under 60 °C;
4. Adding the spirulina fine powder 55mg and the balsam pear fine powder 5.5g into the lychee seed fine powder 10.1g, and evenly mixing the mixture to form 1g/piece troche or 0.5-1g/gian capsule, namely, forming the snacks for balancing non-fasting blood sugar for the middle diabetics in the type II diabetics, wherein, the snacks are taken 5 minutes within the type D breakfast, the lunch and the dinner with warm water under 60 °C;
5. Adding the spirulina fine powder 65mg and the balsam pear fine powder 6g into the lychee seed fine powder 10.3g, and evenly mixing the mixture to form 1g/piece troche or 0.5-1g/gian capsule, namely, forming the snacks for balancing non-fasting blood sugar for the middle diabetics in the type II diabetics, wherein, the snacks are taken 5 minutes within the type E breakfast, the lunch and the dinner with warm water under 60 °C;
6. Adding the spirulina fine powder 75mg and the balsam pear fine powder 6.5g into the lychee seed fine powder 10.5g, and evenly mixing the mixture to form 1g/piece troche or 0.5-1g/gian capsule, namely, forming the snacks for balancing non-fasting blood sugar for the middle diabetics in the type II diabetics, wherein, the snacks are taken 5 minutes within the type F breakfast, the lunch and the dinner with warm water under 60 °C;
7. Adding the spirulina fine powder 85mg and the balsam pear fine powder 7g into the lychee seed fine powder 10.7g, and evenly mixing the mixture to form 1g/piece troche or 0.5-1g/gian capsule, namely, forming the snacks for balancing non-fasting blood sugar for the middle diabetics in the type II diabetics, wherein, the snacks are taken 5 minutes within the type G breakfast, the lunch and the dinner with warm water under 60 °C;
8. Adding the spirulina fine powder 87mg and the balsam pear fine powder 7.5g into the lychee seed fine powder 10.9g, and evenly mixing the mixture to form 1g/piece troche or 0.5-1g/gian capsule, namely, forming the snacks for balancing non-fasting blood sugar for the middle diabetics in the type II diabetics, wherein, the snacks are taken 5 minutes within the type H breakfast, the lunch and the dinner with warm water under 60 °C;.
9. Adding the spirulina fine powder 89mg and the balsam pear fine powder 7.9g into the lychee seed fine powder 11.19g, and evenly mixing the mixture to form 1g/piece troche or 0.5-1g/gian capsule, namely, forming the snacks for balancing non-fasting blood sugar for the middle diabetics in the type II diabetics, wherein, the snacks are taken 5 minutes within the type J breakfast, the lunch and the dinner with warm water under 60 °C;

### Example 7

Aiming at the severe diabetics in the type II diabetics, the preparation, the dosage and the taking mode of snacks for balancing non-fasting blood sugar at every morning are prepared by dosing the prepared lychee seed fine powder, the spirulina fine powder and the balsam pear fine powder under sterile environment in the follows ways:
1. Adding the spirulina fine powder 35mg and the balsam pear fine powder 5g into the lychee seed fine powder 11.3g, and evenly mixing the mixture to form 1g/piece troche or 0.5-1g/gian capsule, namely, forming the snacks for balancing non-fasting blood sugar for the severe diabetics in the type II diabetics, wherein, the snacks are taken 5 minutes within the type A breakfast, the lunch and the dinner with warm water under 60 °C;
2. Adding the spirulina fine powder 45mg and the balsam pear fine powder 5.5g into the lychee seed fine powder 11.5g, and evenly mixing the mixture to form 1g/piece troche or 0.5-1g/gian capsule, namely, forming the snacks for balancing non-fasting blood sugar for the severe diabetics in the type II diabetics, wherein, the snacks are taken 5 minutes within the type B breakfast, the lunch and the dinner with warm water under 60 °C;
3. Adding the spirulina fine powder 55mg and the balsam pear fine powder 6g into the lychee seed fine powder 11.3g, and evenly mixing the mixture to form 1g/piece troche or 0.5-1g/gian capsule, namely, forming the snacks for balancing non-fasting blood sugar for the severe diabetics in the type II diabetics, wherein, the snacks are taken 5 minutes within the type C breakfast, the lunch and the dinner with warm water under 60 °C;
4. Adding the spirulina fine powder 65mg and the balsam pear fine powder 6.5g into the lychee seed fine powder 11.9g, and evenly mixing the mixture to form 1g/piece troche or 0.5-1g/gian capsule, namely, forming the snacks for balancing non-fasting blood sugar for the severe diabetics in the type II diabetics, wherein, the snacks are taken 5 minutes within the type D breakfast, the lunch and the dinner with warm water under 60 °C;
5. Adding the spirulina fine powder 75mg and the balsam pear fine powder 7g into the lychee seed fine powder 12.1g, and evenly mixing the mixture to form 1g/piece troche or 0.5-1g/gian capsule, namely, forming the snacks for balancing non-fasting blood sugar for the severe diabetics in the type II diabetics, wherein, the snacks are taken 5 minutes within the type E breakfast, the lunch and the dinner with warm water under 60 °C;
6. Adding the spirulina fine powder 85mg and the balsam pear fine powder 7.5g into the lychee seed fine powder 12.3g, and evenly mixing the mixture to form 1g/piece troche or 0.5-1g/gian capsule, namely, forming the snacks for balancing non-fasting blood sugar for the severe diabetics in the type II diabetics, wherein, the snacks are taken 5 minutes within the type F breakfast, the lunch and the dinner with warm water under 60 °C;
7. Adding the spirulina fine powder 95mg and the balsam pear fine powder 8g into the lychee seed fine powder 12.5g, and evenly mixing the mixture to form 1g/piece troche or 0.5-1g/gian capsule, namely, forming the snacks for balancing non-fasting blood sugar for the severe diabetics in the type II diabetics, wherein, the snacks are taken 5 minutes within the type G breakfast, the lunch and the dinner with warm water under 60 °C;
8. Adding the spirulina fine powder 97mg and the balsam pear fine powder 8.5g into the lychee seed fine powder 12.7g, and evenly mixing the mixture to form 1g/piece troche or 0.5-1g/gian capsule, namely, forming the snacks for balancing non-fasting blood sugar for the severe diabetics in the type II diabetics, wherein, the snacks are taken 5 minutes within the type A breakfast, the lunch and the dinner with warm water under 60 °C;.
9. Adding the spirulina fine powder 99mg and the balsam pear fine powder 8.9g into the lychee seed fine powder 12.9g, and evenly mixing the mixture to form 1g/piece troche or 0.5-1g/gian capsule, namely, forming the snacks for balancing non-fasting blood sugar for the severe diabetics in the type II diabetics, wherein, the snacks are taken 5 minutes within the type J breakfast, the lunch and the dinner with warm water under 60 °C;

### Example 8

Aiming at the levis diabetics in the type II diabetics, the preparation, the dosage and the taking mode of special snacks which are eaten between the breakfast and the lunch, between the lunch and the dinner as well as 30 minutes before sleep are prepared by dosing the qualifiedly prepared spirulina fine powder, the pumpkin fine powder and the lychee seed fine powder under absolute sterile environment in the follows ways:
1. Adding the pumpkin fine powder 6g and the lychee seed fine powder 5mg into the spirulina fine powder 0.5g, and evenly mixing the mixture to form the snacks with weight of 6.5005g/piece, namely, forming the snacks which are eaten among the type A breakfast, lunch and dinner in 2-3 hours for the levis diabetics in the type II diabetics when the diabetics are hungry;
2. Adding the pumpkin fine powder 6.5g and the lychee seed fine powder 6mg into the spirulina fine powder 0.8g, and evenly mixing the mixture to form the snacks with weight of 7.3006g/piece, namely, forming the snacks which are eaten among the type B breakfast, lunch and dinner in 2-3 hours for the levis diabetics in the type II diabetics when the diabetics are hungry;
3. Adding the pumpkin fine powder 7g and the lychee seed fine powder 7mg into the spirulina fine powder 1.1g, and evenly mixing the mixture to form the snacks with weight of 8.1007g/piece, namely, forming the snacks which are eaten among the type C breakfast, lunch and dinner in 2-3 hours for the levis diabetics in the type II diabetics when the diabetics are hungry;
4. Adding the pumpkin fine powder 7.5g and the lychee seed fine powder 8mg into the spirulina fine powder 1.4g, and evenly mixing the mixture to form the snacks with weight of 8.9008g/piece, namely, forming the snacks which are eaten among the type D breakfast, lunch and dinner in 2-3 hours for the levis diabetics in the type II diabetics when the diabetics are hungry;
5. Adding the pumpkin fine powder 8g and the lychee seed fine powder 9mg into the spirulina fine powder 1.7g, and evenly mixing the mixture to form the snacks with weight of 9.7009g/piece, namely, forming the snacks which are eaten among the type C breakfast, lunch and dinner in 2-3 hours for the levis diabetics in the type II diabetics when the diabetics are hungry;
6. Adding the pumpkin fine powder 8.510.501g and the lychee seed fine powder 10mg into the spirulina fine powder 2g, and evenly mixing the mixture to form the snacks with weight of 16.001g/piece, namely, forming the snacks which are eaten among the type F breakfast, lunch and dinner in 2-3 hours for the levis diabetics in the type II diabetics when the diabetics are hungry;
7. Adding the pumpkin fine powder 9g and the lychee seed fine powder 11mg into the spirulina fine powder 2.3g, and evenly mixing the mixture to form the snacks with weight of 11.3011g/piece, namely, forming the snacks which are eaten among the type G breakfast, lunch and dinner in 2-3 hours for the levis diabetics in the type II diabetics when the diabetics are hungry;
8. Adding the pumpkin fine powder 9.5g and the lychee seed fine powder 11.4mg into the spirulina fine powder 2.5g, and evenly mixing the mixture to form the snacks with weight of 12.0114g/piece, namely, forming the snacks which are eaten among the type H breakfast, lunch and dinner in 2-3 hours for the levis diabetics in the type II diabetics when the diabetics are hungry;
9. Adding the pumpkin fine powder 9.9g and the lychee seed fine powder 11.9mg into the spirulina fine powder 2.7g, and evenly mixing the mixture to form the snacks with weight of 12.6119g/piece, namely, forming the snacks which are eaten among the type J breakfast, lunch and dinner in 2-3 hours for the levis diabetics in the type II diabetics when the diabetics are hungry;

### Example 9

Aiming at the middle diabetics in the type II diabetics, the preparation, the dosage and the taking mode of special snacks which are eaten between the breakfast and the lunch, between the lunch and the dinner as well as 30 minutes before sleep are prepared by dosing the qualifiedly prepared spirulina fine powder, the pumpkin fine powder and the lychee seed fine powder under absolute sterile environment in the follows ways:
1. Adding the pumpkin fine powder 9.5g and the lychee seed fine powder 12mg into the spirulina fine powder 2.6g, and evenly mixing the mixture to form the snacks with weight of 12.1012g/piece, namely, forming the snacks which are eaten among the type A breakfast, lunch and dinner in 2-3 hours for the middle diabetics in the type II diabetics when the diabetics are hungry;
2. Adding the pumpkin fine powder 10g and the lychee seed fine powder 13mg into the spirulina fine powder 2.9g, and evenly mixing the mixture to form the snacks with weight of 12.9013g/piece, namely, forming the snacks which are eaten among the type B breakfast, lunch and dinner in 2-3 hours for the middle diabetics in the type II diabetics when the diabetics are hungry;
3. Adding the pumpkin fine powder 9.5g and the lychee seed fine powder 12mg into the spirulina fine powder 2.6g, and evenly mixing the mixture to form the snacks with weight of 12.1012g/piece, namely, forming the snacks which are eaten among the type C breakfast, lunch and dinner in 2-3 hours for the middle diabetics in the type II diabetics when the diabetics are hungry;
4. Adding the pumpkin fine powder 11g and the lychee seed fine powder 15mg into the spirulina fine powder 3.5g, and evenly mixing the mixture to form the snacks with weight of 14.5015g/piece, namely, forming the snacks which are eaten among the type D breakfast, lunch and dinner in 2-3 hours for the middle diabetics in the type II diabetics when the diabetics are hungry;
5. Adding the pumpkin fine powder 11.5g and the lychee seed fine powder 16mg into the spirulina fine powder 3.8g, and evenly mixing the mixture to form the snacks with weight of 14.3016g/piece, namely, forming the snacks which are eaten among the type E breakfast, lunch and dinner in 2-3 hours for the middle diabetics in the type II diabetics when the diabetics are hungry;
6. Adding the pumpkin fine powder 12g and the lychee seed fine powder 17mg into the spirulina fine powder 4.1g, and evenly mixing the mixture to form the snacks with weight of 16.1017g/piece, namely, forming the snacks which are eaten among the type F breakfast, lunch and dinner in 2-3 hours for the middle diabetics in the type II diabetics when the diabetics are hungry;
7. Adding the pumpkin fine powder 12.5g and the lychee seed fine powder 18mg into the spirulina fine powder 4.4g, and evenly mixing the mixture to form the snacks with weight of 16.9018g/piece, namely, forming the snacks which are eaten among the type G breakfast, lunch and dinner in 2-3 hours for the middle diabetics in the type II diabetics when the diabetics are hungry;
8. Adding the pumpkin fine powder 12.7g and the lychee seed fine powder 18.4mg into the spirulina fine powder 4.6g, and evenly mixing the mixture to form the snacks with weight of 12.7g/piece, namely, forming the snacks which are eaten among the type H breakfast, lunch and dinner in 2-3 hours for the middle diabetics in the type II diabetics when the diabetics are hungry;
9. Adding the pumpkin fine powder 12.9g and the lychee seed fine powder 18.8mg into the spirulina fine powder 4.8g, and evenly mixing the mixture to form the snacks with weight of 17.7188g/piece, namely, forming the snacks which are eaten among the type G breakfast, lunch and dinner in 2-3 hours for the middle diabetics in the type II diabetics when the diabetics are hungry;

### Example 10

Aiming at the severe diabetics in the type II diabetics, the preparation, the dosage and the taking mode of special snacks which are eaten between the breakfast and the lunch, between the lunch and the dinner as well as 30 minutes before sleep are prepared by dosing the qualifiedly prepared spirulina fine powder, the pumpkin fine powder and the lychee seed fine powder under absolute sterile environment in the follows ways:
1. Adding the pumpkin fine powder 13g and the lychee seed fine powder 19mg into the spirulina fine powder 4.5g, and evenly mixing the mixture to form the snacks with weight of 17.5019g/piece, namely, forming the snacks which are eaten among the type A breakfast, lunch and dinner in 2-3 hours for the levis diabetics in the type II diabetics when the diabetics are hungry;
2. Adding the pumpkin fine powder 13.5g and the lychee seed fine powder 20mg into the spirulina fine powder 4.6g, and evenly mixing the mixture to form the snacks with weight of 18.102g/piece, namely, forming the snacks which are eaten among the type B breakfast, lunch and dinner in 2-3 hours for the levis diabetics in the type II diabetics when the diabetics are hungry;
3. Adding the pumpkin fine powder 14g and the lychee seed fine powder 21mg into the spirulina fine powder 4.7g, and evenly mixing the mixture to form the snacks with weight of 18.7021g/piece, namely, forming the snacks which are eaten among the type C breakfast, lunch and dinner in 2-3 hours for the levis diabetics in the type II diabetics when the diabetics are hungry;
4. Adding the pumpkin fine powder 14.5g and the lychee seed fine powder 22mg into the spirulina fine powder 4.8g, and evenly mixing the mixture to form the snacks with weight of 18.3022g/piece, namely, forming the snacks which are eaten among the type D breakfast, lunch and dinner in 2-3 hours for the levis diabetics in the type II diabetics when the diabetics are hungry;
5. Adding the pumpkin fine powder 15g and the lychee seed fine powder 23mg into the spirulina fine powder 4.9g, and evenly mixing the mixture to form the snacks with weight of 19.9023g/piece, namely, forming the snacks which are eaten among the type E breakfast, lunch and dinner in 2-3 hours for the levis diabetics in the type II diabetics when the diabetics are hungry;
6. Adding the pumpkin fine powder 15.5g and the lychee seed fine powder 24mg into the spirulina fine powder 5g, and evenly mixing the mixture to form the snacks with weight of 20.5024g/piece, namely, forming the snacks which are eaten among the type F breakfast, lunch and dinner in 2-3 hours for the levis diabetics in the type II diabetics when the diabetics are hungry;
7. Adding the pumpkin fine powder 16g and the lychee seed fine powder 25mg into the spirulina fine powder 4.5g, and evenly mixing the mixture to form the snacks with weight of 21.1025g/piece, namely, forming the snacks which are eaten among the type G breakfast, lunch and dinner in 2-3 hours for the levis diabetics in the type II diabetics when the diabetics are hungry;
8. Adding the pumpkin fine powder 16.5g and the lychee seed fine powder 26mg into the spirulina fine powder 5.25g, and evenly mixing the mixture to form the snacks with weight of 21.7026g/piece, namely, forming the snacks which are eaten among the type H breakfast, lunch and dinner in 2-3 hours for the levis diabetics in the type II diabetics when the diabetics are hungry;
9. Adding the pumpkin fine powder 17g and the lychee seed fine powder 27mg into the spirulina fine powder 5.3g, and evenly mixing the mixture to form the snacks with weight of 22.3027g/piece, namely, forming the snacks which are eaten among the type J breakfast, lunch and dinner in 2-3 hours for the levis diabetics in the type II diabetics when the diabetics are hungry;

### Example 11

Aiming at the levis diabetics in the type II diabetics, the preparation, the dosage and the taking mode of special snacks which have to be eaten before sleep for balancing night blood sugar are prepared by dosing the qualifiedly prepared standard spirulina fine powder, the pumpkin fine powder, the balsam pear fine powder and the lychee seed fine powder under absolute sterile environment in the follows ways:
1. Adding the pumpkin fine powder 2.5g, the balsam pear fine powder 2mg and the lychee seed fine powder 2mg into the spirulina fine powder 1.5g, and evenly mixing the mixture to form x4 grains with equal weight, namely, forming the snacks which are eaten after the type A dinner and 10 minutes before sleep for the levis diabetics in the type II diabetics, wherein, the snacks have to be eaten at one time for non-fasting food therapy before the sleep with warmer water under 60 °C;
2. Adding the pumpkin fine powder 3.0g, the balsam pear fine powder 4mg and the lychee seed fine powder 2mg into the spirulina fine powder 2.0g, and evenly mixing the mixture to form x4 grains with equal weight, namely, forming the snacks which are eaten after the type B dinner and 10 minutes before sleep for the levis diabetics in the type II diabetics, wherein, the snacks have to be eaten at one time for non-fasting food therapy before the sleep with warmer water under 60 °C;
3. Adding the pumpkin fine powder 3.5g, the balsam pear fine powder 6mg and the lychee seed fine powder 2mg into the spirulina fine powder 2.5g, and evenly mixing the mixture to form x4 grains with equal weight, namely, forming the snacks which are eaten after the type C dinner and 10 minutes before sleep for the levis diabetics in the type II diabetics, wherein, the snacks have to be eaten at one time for non-fasting food therapy before the sleep with warmer water under 60 °C;
4. Adding the pumpkin fine powder 4.0g, the balsam pear fine powder 8mg and the lychee seed fine powder 2mg into the spirulina fine powder 3.0g, and evenly mixing the mixture to form x4 grains with equal weight, namely, forming the snacks which are eaten after the type D dinner and 10 minutes before sleep for the levis diabetics in the type II diabetics, wherein, the snacks have to be eaten at one time for non-fasting food therapy before the sleep with warmer water under 60 °C;
5. Adding the pumpkin fine powder 4.5g, the balsam pear fine powder 10mg and the lychee seed fine powder 2mg into the spirulina fine powder 3.5g, and evenly mixing the mixture to form x4 grains with equal weight, namely, forming the snacks which are eaten after the type E dinner and 10 minutes before sleep for the levis diabetics in the type II diabetics, wherein, the snacks have to be eaten at one time for non-fasting food therapy before the sleep with warmer water under 60 °C;
6. Adding the pumpkin fine powder 5.0g, the balsam pear fine powder 12mg and the lychee seed fine powder 2mg into the spirulina fine powder 4.0g, and evenly mixing the mixture to form x4 grains with equal weight, namely, forming the snacks which are eaten after the type F dinner and 10 minutes before sleep for the levis diabetics in the type II diabetics, wherein, the snacks have to be eaten at one time for non-fasting food therapy before the sleep with warmer water under 60 °C;
7. Adding the pumpkin fine powder 5.5g, the balsam pear fine powder 14mg and the lychee seed fine powder 2mg into the spirulina fine powder 4.5g, and evenly mixing the mixture to form x4 grains with equal weight, namely, forming the snacks which are eaten after the type G dinner and 10 minutes before sleep for the levis diabetics in the type II diabetics, wherein, the snacks have to be eaten at one time for non-fasting food therapy before the sleep with warmer water under 60 °C;
8. Adding the pumpkin fine powder 5.5g, the balsam pear fine powder 15mg and the lychee seed fine powder 2mg into the spirulina fine powder 4.7g, and evenly mixing the mixture to form x4 grains with equal weight, namely, forming the snacks which are eaten after the type G dinner and 10 minutes before sleep for the levis diabetics in the type II diabetics, wherein, the snacks have to be eaten at one time for non-fasting food therapy before the sleep with warmer water under 60 °C;
9. Adding the pumpkin fine powder 5.5g, the balsam pear fine powder 16mg and the lychee seed fine powder 2mg into the spirulina fine powder 4.9g, and evenly mixing the mixture to form x4 grains with equal weight, namely, forming the snacks which are eaten after the type J dinner and 10 minutes before sleep for the levis diabetics in the type II diabetics, wherein, the snacks have to be eaten at one time for non-fasting food therapy before the sleep with warmer water under 60 °C;

### Example 12

Aiming at the middle diabetics in the type II diabetics, the preparation, the dosage and the taking mode of special snacks which have to be eaten before sleep for balancing night blood sugar are prepared by dosing the qualifiedly prepared standard spirulina fine powder, the pumpkin fine powder, the balsam pear fine powder and the lychee seed fine powder under absolute sterile environment in the follows ways:
1. Adding the pumpkin fine powder 3.5g, the balsam pear fine powder 14.1mg and the lychee seed fine powder 2mg into the spirulina fine powder 5g, and evenly mixing the mixture to form x5 grains with equal weight, namely, forming the snacks which are eaten after the type A dinner and 10 minutes before sleep for the middle diabetics in the type II diabetics, wherein, the snacks have to be eaten at one time for non-fasting food therapy before the sleep with warmer water under 60 °C;
2. Adding the pumpkin fine powder 4.0g, the balsam pear fine powder 14.2mg and the lychee seed fine powder 2mg into the spirulina fine powder 5.1g, and evenly mixing the mixture to form x5 grains with equal weight, namely, forming the snacks which are eaten after the type B dinner and 10 minutes before sleep for the middle diabetics in the type II diabetics, wherein, the snacks have to be eaten at one time for non-fasting food therapy before the sleep with warmer water under 60 °C;
3. Adding the pumpkin fine powder 5.5g, the balsam pear fine powder 14.3mg and the lychee seed fine powder 2mg into the spirulina fine powder 5.3g, and evenly mixing the mixture to form x5 grains with equal weight, namely, forming the snacks which are eaten after the type C dinner and 10 minutes before sleep for the middle diabetics in the type II diabetics, wherein, the snacks have to be eaten at one time for non-fasting food therapy before the sleep with warmer water under 60 °C;
4. Adding the pumpkin fine powder 6.0g, the balsam pear fine powder 14.4mg and the lychee seed fine powder 2mg into the spirulina fine powder 5.5g, and evenly mixing the mixture to form x5 grains with equal weight, namely, forming the snacks which are eaten after the type D dinner and 10 minutes before sleep for the middle diabetics in the type II diabetics, wherein, the snacks have to be eaten at one time for non-fasting food therapy before the sleep with warmer water under 60 °C;
5. Adding the pumpkin fine powder 7.5g, the balsam pear fine powder 14.5mg and the lychee seed fine powder 2mg into the spirulina fine powder 5.7g, and evenly mixing the mixture to form x5 grains with equal weight, namely, forming the snacks which are eaten after the type E dinner and 10 minutes before sleep for the middle diabetics in the type II diabetics, wherein, the snacks have to be eaten at one time for non-fasting food therapy before the sleep with warmer water under 60 °C;
6. Adding the pumpkin fine powder 8.0g, the balsam pear fine powder 14.6mg and the lychee seed fine powder 2mg into the spirulina fine powder 5.9g, and evenly mixing the mixture to form x5 grains with equal weight, namely, forming the snacks which are eaten after the type F dinner and 10 minutes before sleep for the middle diabetics in the type II diabetics, wherein, the snacks have to be eaten at one time for non-fasting food therapy before the sleep with warmer water under 60 °C;
7. Adding the pumpkin fine powder 9.5g, the balsam pear fine powder 14.7mg and the lychee seed fine powder 2mg into the spirulina fine powder 6.1g, and evenly mixing the mixture to form x5 grains with equal weight, namely, forming the snacks which are eaten after the type G dinner and 10 minutes before sleep for the middle diabetics in the type II diabetics, wherein, the snacks have to be eaten at one time for non-fasting food therapy before the sleep with warmer water under 60 °C;
8. Adding the pumpkin fine powder 9.5g, the balsam pear fine powder 14.8mg and the lychee seed fine powder 2mg into the spirulina fine powder 6.2g, and evenly mixing the mixture to form x5 grains with equal weight, namely, forming the snacks which are eaten after the type H dinner and 10 minutes before sleep for the middle diabetics in the type II diabetics, wherein, the snacks have to be eaten at one time for non-fasting food therapy before the sleep with warmer water under 60 °C;
9. Adding the pumpkin fine powder 9.5g, the balsam pear fine powder 14.9mg and the lychee seed fine powder 2mg into the spirulina fine powder 6.3g, and evenly mixing the mixture to form x5 grains with equal weight, namely, forming the snacks which are eaten after the type J dinner and 10 minutes before sleep for the middle diabetics in the type II diabetics, wherein, the snacks have to be eaten at one time for non-fasting food therapy before the sleep with warmer water under 60 °C;

### Example 13

Aiming at the severe diabetics in the type II diabetics, the preparation, the dosage and the taking mode of special snacks which have to be eaten before sleep for balancing night blood sugar are prepared by dosing the qualifiedly prepared standard spirulina fine powder, the pumpkin fine powder, the balsam pear fine powder and the lychee seed fine powder under absolute sterile environment in the follows ways:
1. Adding the pumpkin fine powder 5.5g, the balsam pear fine powder 15mg and the lychee seed fine powder 2mg into the spirulina fine powder 6.4g, and evenly mixing the mixture to form x6 grains with equal weight, namely, forming the snacks which are eaten after the type A dinner and 10 minutes before sleep for the middle diabetics in the type II diabetics, wherein, the snacks have to be eaten at one time for non-fasting food therapy before the sleep with warmer water under 60 °C;
2. Adding the pumpkin fine powder 6.0g, the balsam pear fine powder 15.1mg and the lychee seed fine powder 2mg into the spirulina fine powder 6.5g, and evenly mixing the mixture to form x6 grains with equal weight, namely, forming the snacks which are eaten after the type B dinner and 10 minutes before sleep for the middle diabetics in the type II diabetics, wherein, the snacks have to be eaten at one time for non-fasting food therapy before the sleep with warmer water under 60 °C;
3. Adding the pumpkin fine powder 7.5g, the balsam pear fine powder 15.2mg and the lychee seed fine powder 2mg into the spirulina fine powder 6.6g, and evenly mixing the mixture to form x6 grains with equal weight, namely, forming the snacks which are eaten after the type C dinner and 10 minutes before sleep for the middle diabetics in the type II diabetics, wherein, the snacks have to be eaten at one time for non-fasting food therapy before the sleep with warmer water under 60 °C;
4. Adding the pumpkin fine powder 9.0g, the balsam pear fine powder 15.3mg and the lychee seed fine powder 2mg into the spirulina fine powder 6.7g, and evenly mixing the mixture to form x6 grains with equal weight, namely, forming the snacks which are eaten after the type D dinner and 10 minutes before sleep for the middle diabetics in the type II diabetics, wherein, the snacks have to be eaten at one time for non-fasting food therapy before the sleep with warmer water under 60 °C;
5. Adding the pumpkin fine powder 10.5g, the balsam pear fine powder 15.4mg and the lychee seed fine powder 2mg into the spirulina fine powder 6.8g, and evenly mixing the mixture to form x6 grains with equal weight, namely, forming the snacks which are eaten after the type E dinner and 10 minutes before sleep for the middle diabetics in the type II diabetics, wherein, the snacks have to be eaten at one time for non-fasting food therapy before the sleep with warmer water under 60 °C;
6. Adding the pumpkin fine powder 11.0g, the balsam pear fine powder 15.5mg and the lychee seed fine powder 2mg into the spirulina fine powder 6.9g, and evenly mixing the mixture to form x6 grains with equal weight, namely, forming the snacks which are eaten after the type F dinner and 10 minutes before sleep for the middle diabetics in the type II diabetics, wherein, the snacks have to be eaten at one time for non-fasting food therapy before the sleep with warmer water under 60 °C;
7. Adding the pumpkin fine powder 12.5g, the balsam pear fine powder 15.6mg and the lychee seed fine powder 2mg into the spirulina fine powder 7.0g, and evenly mixing the mixture to form x6 grains with equal weight, namely, forming the snacks which are eaten after the type G dinner and 10 minutes before sleep for the middle diabetics in the type II diabetics, wherein, the snacks have to be eaten at one time for non-fasting food therapy before the sleep with warmer water under 60 °C;
8. Adding the pumpkin fine powder 12.7g, the balsam pear fine powder 15.7mg and the lychee seed fine powder 2mg into the spirulina fine powder 7.1g, and evenly mixing the mixture to form x6 grains with equal weight, namely, forming the snacks which are eaten after the type H dinner and 10 minutes before sleep for the middle diabetics in the type II diabetics, wherein, the snacks have to be eaten at one time for non-fasting food therapy before the sleep with warmer water under 60 °C;
9. Adding the pumpkin fine powder 12.9g, the balsam pear fine powder 15.8mg and the lychee seed fine powder 2mg into the spirulina fine powder 7.2g, and evenly mixing the mixture to form x6 grains with equal weight, namely, forming the snacks which are eaten after the type J dinner and 10 minutes before sleep for the middle diabetics in the type II diabetics, wherein, the snacks have to be eaten at one time for non-fasting food therapy before the sleep with warmer water under 60 °C;

### Example 14

Aiming at the levis diabetics in the type II diabetics complicating with Hyperlipidemia and hyperpiesis, the preparation, the dosage and the taking mode of special snacks which have to be eaten before sleep at the morning, the noon and the night are prepared by dosing the qualifiedly prepared standard spirulina fine powder and the balsam pear fine powder under absolute sterile environment in the follows ways:
1. Adding the balsam pear fine powder 4g into the spirulina fine powder 1.1g, and evenly mixing the mixture to form 4 grains with equal weight or capsule with equal weight, namely, forming the snacks which are eaten before type A breakfast, lunch, dinner and 10 minutes before sleep at each day for the levis diabetics in the type II diabetics complicating with hyperlipidemia and hyperpiesis, wherein, the snacks which are used for reducing blood fat and blood pressure have to be eaten at one time for fasting food therapy with warmer water under 60 °C;
2. Adding the balsam pear fine powder 4.3g into the spirulina fine powder 1.2g, and evenly mixing the mixture to form 4 grains with equal weight or capsule with equal weight, namely, forming the snacks which are eaten before type B breakfast, lunch, dinner and 10 minutes before sleep at each day for the levis diabetics in the type II diabetics complicating with hyperlipidemia and hyperpiesis, wherein, the snacks which are used for reducing blood fat and blood pressure have to be eaten at one time for fasting food therapy with warmer water under 60 °C;
3. Adding the balsam pear fine powder 4.6g into the spirulina fine powder 1.3g, and evenly mixing the mixture to form 4 grains with equal weight or capsule with equal weight, namely, forming the snacks which are eaten before type C breakfast, lunch, dinner and 10 minutes before sleep at each day for the levis diabetics in the type II diabetics complicating with hyperlipidemia and hyperpiesis, wherein, the snacks which are used for reducing blood fat and blood pressure have to be eaten at one time for fasting food therapy with warmer water under 60 °C;
4. Adding the balsam pear fine powder 4.9g into the spirulina fine powder 1.4g, and evenly mixing the mixture to form 4 grains with equal weight, namely, forming the snacks which are eaten before type D breakfast, lunch, dinner and 10 minutes before sleep at each day for the levis diabetics in the type II diabetics complicating with hyperlipidemia and hyperpiesis, wherein, the snacks which are used for reducing blood fat and blood pressure have to be eaten at one time for fasting food therapy with warmer water under 60 °C;
5. Adding the balsam pear fine powder 5.2g into the spirulina fine powder 1.5g, and evenly mixing the mixture to form 4 grains with equal weight, namely, forming the snacks which are eaten before type E breakfast, lunch, dinner and 10 minutes before sleep at each day for the levis diabetics in the type II diabetics complicating with hyperlipidemia and hyperpiesis, wherein, the snacks which are used for reducing blood fat and blood pressure have to be eaten at one time for fasting food therapy with warmer water under 60 °C;
6. Adding the balsam pear fine powder 5.5g into the spirulina fine powder 1.4g, and evenly mixing the mixture to form 4 grains with equal weight, namely, forming the snacks which are eaten before type F breakfast, lunch, dinner and 10 minutes before sleep at each day for the levis diabetics in the type II diabetics complicating with hyperlipidemia and hyperpiesis, wherein, the snacks which are used for reducing blood fat and blood pressure have to be eaten at one time for fasting food therapy with warmer water under 60 °C;
7. Adding the balsam pear fine powder 5.8g into the spirulina fine powder 1.7g, and evenly mixing the mixture to form 4 grains with equal weight, namely, forming the snacks which are eaten before type G breakfast, lunch, dinner and 10 minutes before sleep at each day for the levis diabetics in the type II diabetics complicating with hyperlipidemia and hyperpiesis, wherein, the snacks which are used for reducing blood fat and blood pressure have to be eaten at one time for fasting food therapy with warmer water under 60 °C;
8. Adding the balsam pear fine powder 6.0g into the spirulina fine powder 1.8g, and evenly mixing the mixture to form 4 grains with equal weight, namely, forming the snacks which are eaten before type H breakfast, lunch, dinner and 10 minutes before sleep at each day for the levis diabetics in the type II diabetics complicating with hyperlipidemia and hyperpiesis, wherein, the snacks which are used for reducing blood fat and blood pressure have to be eaten at one time for fasting food therapy with warmer water under 60 °C;
9. Adding the balsam pear fine powder 6.1g into the spirulina fine powder 1.9g, and evenly mixing the mixture to form 4 grains with equal weight, namely, forming the snacks which are eaten before type J breakfast, lunch, dinner and 10 minutes before sleep at each day for the levis diabetics in the type II diabetics complicating with hyperlipidemia and hyperpiesis, wherein, the snacks which are used for reducing blood fat and blood pressure have to be eaten at one time for fasting food therapy with warmer water under 60 °C;

### Example 15

Aiming at the middle diabetics in the type II diabetics complicating with Hyperlipidemia and hyperpiesis, the preparation, the dosage and the taking mode of special snacks which are eaten before sleep at the morning, the noon and the night are prepared by dosing the qualifiedly prepared standard spirulina fine powder and the balsam pear fine powder under absolute sterile environment in the follows ways:
1. Adding the balsam pear fine powder 4g into the spirulina fine powder 1.1g, and evenly mixing the mixture to form 5 grains with equal weight, namely, forming the snacks which are eaten before type A breakfast, lunch, dinner and 10 minutes before sleep at each day for the middle diabetics in the type II diabetics complicating with hyperlipidemia and hyperpiesis, wherein, the snacks which are used for reducing blood fat and blood pressure have to be eaten at one time for fasting food therapy with warmer water under 60 °C;
2. Adding the balsam pear fine powder 6.3g into the spirulina fine powder 2.1g, and evenly mixing the mixture to form 5 grains with equal weight, namely, forming the snacks which are eaten before type B breakfast, lunch, dinner and 10 minutes before sleep at each day for the middle diabetics in the type II diabetics complicating with hyperlipidemia and hyperpiesis, wherein, the snacks which are used for reducing blood fat and blood pressure have to be eaten at one time for fasting food therapy with warmer water under 60 °C;
3. Adding the balsam pear fine powder 6.4g into the spirulina fine powder 2.2g, and evenly mixing the mixture to form 5 grains with equal weight, namely, forming the snacks which are eaten before type C breakfast, lunch, dinner and 10 minutes before sleep at each day for the middle diabetics in the type II diabetics complicating with hyperlipidemia and hyperpiesis, wherein, the snacks which are used for reducing blood fat and blood pressure have to be eaten at one time for fasting food therapy with warmer water under 60 °C;
4. Adding the balsam pear fine powder 6.5g into the spirulina fine powder 2.3g, and evenly mixing the mixture to form 5 grains with equal weight, namely, forming the snacks which are eaten before type D breakfast, lunch, dinner and 10 minutes before sleep at each day for the middle diabetics in the type II diabetics complicating with hyperlipidemia and hyperpiesis, wherein, the snacks which are used for reducing blood fat and blood pressure have to be eaten at one time for fasting food therapy with warmer water under 60 °C;
5. Adding the balsam pear fine powder 6.6g into the spirulina fine powder 2.4g, and evenly mixing the mixture to form 5 grains with equal weight, namely, forming the snacks which are eaten before type E breakfast, lunch, dinner and 10 minutes before sleep at each day for the middle diabetics in the type II diabetics complicating with hyperlipidemia and hyperpiesis, wherein, the snacks which are used for reducing blood fat and blood pressure have to be eaten at one time for fasting food therapy with warmer water under 60 °C;
6. Adding the balsam pear fine powder 6.7g into the spirulina fine powder 2.5g, and evenly mixing the mixture to form 5 grains with equal weight, namely, forming the snacks which are eaten before type F breakfast, lunch, dinner and 10 minutes before sleep at each day for the middle diabetics in the type II diabetics complicating with hyperlipidemia and hyperpiesis, wherein, the snacks which are used for reducing blood fat and blood pressure have to be eaten at one time for fasting food therapy with warmer water under 60 °C;
7. Adding the balsam pear fine powder 6.8g into the spirulina fine powder 2.6g, and evenly mixing the mixture to form 5 grains with equal weight, namely, forming the snacks which are eaten before type G breakfast, lunch, dinner and 10 minutes before sleep at each day for the middle diabetics in the type II diabetics complicating with hyperlipidemia and hyperpiesis, wherein, the snacks which are used for reducing blood fat and blood pressure have to be eaten at one time for fasting food therapy with warmer water under 60 °C;
8. Adding the balsam pear fine powder 6.9g into the spirulina fine powder 2.7g, and evenly mixing the mixture to form 5 grains with equal weight, namely, forming the snacks which are eaten before type H breakfast, lunch, dinner and 10 minutes before sleep at each day for the middle diabetics in the type II diabetics complicating with hyperlipidemia and hyperpiesis, wherein, the snacks which are used for reducing blood fat and blood pressure have to be eaten at one time for fasting food therapy with warmer water under 60 °C;
9. Adding the balsam pear fine powder 7.0g into the spirulina fine powder 2.8g, and evenly mixing the mixture to form 5 grains with equal weight, namely, forming the snacks which are eaten before type J breakfast, lunch, dinner and 10 minutes before sleep at each day for the middle diabetics in the type II diabetics complicating with hyperlipidemia and hyperpiesis, wherein, the snacks which are used for reducing blood fat and blood pressure have to be eaten at one time for fasting food therapy with warmer water under 60 °C;

### Example 16

Aiming at the severe diabetics in the type II diabetics complicating with Hyperlipidemia and hyperpiesis, the preparation, the dosage and the taking mode of special snacks which are eaten before sleep at the morning, the noon and the night are prepared by dosing the qualifiedly prepared standard spirulina fine powder and the balsam pear fine powder under absolute sterile environment in the follows ways:
1. Adding the balsam pear fine powder 7.1g into the spirulina fine powder 2.9g, and evenly mixing the mixture to form 6 grains with equal weight, namely, forming the snacks which are eaten before type A breakfast, lunch, dinner and 10 minutes before sleep at each day for the severe diabetics in the type II diabetics complicating with hyperlipidemia and hyperpiesis, wherein, the snacks which are used for reducing blood fat and blood pressure have to be eaten at one time for fasting food therapy with warmer water under 60 °C;
2. Adding the balsam pear fine powder 7.2g into the spirulina fine powder 3.0g, and evenly mixing the mixture to form 6 grains with equal weight, namely, forming the snacks which are eaten before type B breakfast, lunch, dinner and 10 minutes before sleep at each day for the severe diabetics in the type II diabetics complicating with hyperlipidemia and hyperpiesis, wherein, the snacks which are used for reducing blood fat and blood pressure have to be eaten at one time for fasting food therapy with warmer water under 60 °C;
3. Adding the balsam pear fine powder 7.3g into the spirulina fine powder 3.1g, and evenly mixing the mixture to form 6 grains with equal weight, namely, forming the snacks which are eaten before type C breakfast, lunch, dinner and 10 minutes before sleep at each day for the severe diabetics in the type II diabetics complicating with hyperlipidemia and hyperpiesis, wherein, the snacks which are used for reducing blood fat and blood pressure have to be eaten at one time for fasting food therapy with warmer water under 60 °C;
4. Adding the balsam pear fine powder 7.4g into the spirulina fine powder 3.2g, and evenly mixing the mixture to form 6 grains with equal weight, namely, forming the snacks which are eaten before type D breakfast, lunch, dinner and 10 minutes before sleep at each day for the severe diabetics in the type II diabetics complicating with hyperlipidemia and hyperpiesis, wherein, the snacks which are used for reducing blood fat and blood pressure have to be eaten at one time for fasting food therapy with warmer water under 60 °C;
5. Adding the balsam pear fine powder 7.5g into the spirulina fine powder 3.3g, and evenly mixing the mixture to form 6 grains with equal weight, namely, forming the snacks which are eaten before type E breakfast, lunch, dinner and 10 minutes before sleep at each day for the severe diabetics in the type II diabetics complicating with hyperlipidemia and hyperpiesis, wherein, the snacks which are used for reducing blood fat and blood pressure have to be eaten at one time for fasting food therapy with warmer water under 60 °C;
6. Adding the balsam pear fine powder 7.6g into the spirulina fine powder 3.4g, and evenly mixing the mixture to form 6 grains with equal weight, namely, forming the snacks which are eaten before type F breakfast, lunch, dinner and 10 minutes before sleep at each day for the severe diabetics in the type II diabetics complicating with hyperlipidemia and hyperpiesis, wherein, the snacks which are used for reducing blood fat and blood pressure have to be eaten at one time for fasting food therapy with warmer water under 60 °C;
7. Adding the balsam pear fine powder 7.7g into the spirulina fine powder 3.5g, and evenly mixing the mixture to form 6 grains with equal weight, namely, forming the snacks which are eaten before type G breakfast, lunch, dinner and 10 minutes before sleep at each day for the severe diabetics in the type II diabetics complicating with hyperlipidemia and hyperpiesis, wherein, the snacks which are used for reducing blood fat and blood pressure have to be eaten at one time for fasting food therapy with warmer water under 60 °C;
8. Adding the balsam pear fine powder 7.8g into the spirulina fine powder 3.6g, and evenly mixing the mixture to form 6 grains with equal weight, namely, forming the snacks which are eaten before type H breakfast, lunch, dinner and 10 minutes before sleep at each day for the severe diabetics in the type II diabetics complicating with hyperlipidemia and hyperpiesis, wherein, the snacks which are used for reducing blood fat and blood pressure have to be eaten at one time for fasting food therapy with warmer water under 60 °C;
9. Adding the balsam pear fine powder 7.9g into the spirulina fine powder 3.7g, and evenly mixing the mixture to form 6 grains with equal weight, namely, forming the snacks which are eaten before type J breakfast, lunch, dinner and 10 minutes before sleep at each day for the severe diabetics in the type II diabetics complicating with hyperlipidemia and hyperpiesis, wherein, the snacks which are used for reducing blood fat and blood pressure have to be eaten at one time for fasting food therapy with warmer water under 60 °C;

The prescription and the use of the present invention effectively reduce and stabilize the blood sugar concentration of the diabetics, avoid the complicating diseases of the diabetics, and ensure the health of the patients.

## Claims

1. A composition for use in treating diabetes comprising the following active components based on the parts by weight:
10-100 parts of Pumpkin powder;
6-200 parts of spirulina; and
2-90 parts of Lychee Seed.

2. The composition for use according to claim 1, wherein the composition further comprises 10-100 parts of balsam pear powder.

3. The composition for use according to claim 2, wherein the composition comprises the following active components based on the parts by weight:
10-60 parts of Pumpkin powder;
6-80 parts of spirulina;
2-60 parts of Lychee Seed; and
10-60 parts of balsam pear powder.

4. A method for preparing the composition for use according to any one of claims 1-3 comprising steps of grinding and mixing the active components.

5. Use of the composition of any one of claims 1-3 for preparing the medicament for treating the diabetes.

## Patentansprüche

1. Zusammensetzung zur Verwendung bei der Behandlung von Diabetes, die die folgenden Wirkbestandteile umfasst, bezogen auf die Gewichtsteile:
10 - 100 Teile Kürbispulver;
6 - 200 Teile Spirulina und
2 - 90 Teile Litschisamen.

2. Zusammensetzung zur Verwendung nach Anspruch 1, wobei die Zusammensetzung weiterhin 10 - 100 Teile Bittermelonenpulver umfasst.

3. Zusammensetzung zur Verwendung nach Anspruch 2, wobei die Zusammensetzung die folgenden Wirkbestandteile umfasst, bezogen auf die Gewichtsteile:
10 - 60 Teile Kürbispulver;
6 - 80 Teile Spirulina;
2 - 60 Teile Litschisamen und
10 - 60 Teile Bittermelonenpulver.

4. Verfahren zur Herstellung der Zusammensetzung zur Verwendung nach einem der Ansprüche 1 - 3, das Schritte des Mahlens und Mischens der Wirkbestandteile umfasst.

5. Verwendung der Zusammensetzung nach einem der Ansprüche 1 - 3 zur Herstellung des Arzneimittels zur Behandlung des Diabetes.

## Revendications

1. Composition pour une utilisation dans le traitement du diabète, comprenant les principes actifs suivants, en parties en poids :
10 à 100 parties de poudre de citrouille ;
6 à 200 parties de spiruline ; et
2 à 90 parties de graines de litchi.

2. Composition pour une utilisation selon la revendication 1, la composition comprenant en outre 10 à 100 parties de poudre de margose.

3. Composition pour une utilisation selon la revendication 2, la composition comprenant les principes actifs suivants, en parties en poids :
10 à 60 parties de poudre de citrouille ;
6 à 80 parties de spiruline ;
2 à 60 parties de graines de litchi ; et
10 à 60 parties de poudre de margose.

4. Procédé de préparation de la composition pour une utilisation selon l'une quelconque des revendications 1 à 3, comprenant les étapes de broyage et de mélange des principes actifs.

5. Utilisation de la composition selon l'une quelconque des revendications 1 à 3, pour la préparation du médicament pour le traitement du diabète.
